# EUROPEAN PATENT SPECIFICATION

(11) **EP 4 077 285 B1**
(45) Date of publication and mention of the grant of the patent: **31.12.2025**
(21) Application number: 20820958.5
(22) Date of filing: 11.12.2020
(51) Int. Cl.: C07D 261/04

(54) **PROCESS FOR PREPARING FLURALANER**
VERFAHREN ZUR HERSTELLUNG VON FLURALANER
PROCÉDÉ DE PRÉPARATION DU FLURALANER

(30) Priority: 17.12.2019 EP 19217100
(43) Date of publication of application: 26.10.2022
(73) Proprietor: KRKA, d.d., Novo mesto, 8501 Novo mesto (SI)
(72) Inventor: RUZIC, Milos, 8501 Novo mesto (SI); TESTEN, Ana, 8501 Novo mesto (SI); PLEVNIK, Miha, 8501 Novo mesto (SI); KLOBCAR, Andrej, 8501 Novo mesto (SI); BENEDIK, Milena, 8501 Novo mesto (SI); MAJCEN, Slavica, 8501 Novo mesto (SI); CANIC NOVAK, Peter, 8501 Novo mesto (SI)
(74) Representative: Kutzenberger Wolff & Partner
(86) International application number: PCT/EP2020/085696
(87) International publication number: WO 2021/122356

(56) References cited:
- EP-A1- 1 731 512
- ERIC VALEUR ET AL: "Amide bond formation: beyond the myth of coupling reagents", CHEMICAL SOCIETY REVIEWS, vol. 38, no. 2, 1 January 2009 (2009-01-01), pages 606, XP055025820, ISSN: 0306-0012, DOI: 10.1039/B701677H
- SIRILAK WANGNGAE ET AL: "Significance of reagent addition sequence in the amidation of carboxylic acids mediated by PPh 3 and I 2", RSC ADVANCES, vol. 5, no. 33, 1 January 2015 (2015-01-01), pages 25789 - 25793, XP055682855, DOI: 10.1039/C5RA03184B

## Description

Priority is claimed of European patent application no. 19 217 100, filed on December 17, 2019.

The invention relates to an improved process for the synthesis of Fluralaner in an efficient and economical way.

Fluralaner is a compound having the chemical name 4-[(5R/S)-5-(3,5-Dichlorophenyl)-4,5-dihydro-5-trifluoromethyl-1,2-oxazol-3-yl]-N-[2-oxo-2-(2,2,2-trifluoroethylamino)ethyl]-o-toluamide or 4-[5-(3,5-dichlorophenyl)-4,5-dihydro-5-(trifluoromethyl)-3-isoxazolyl]-2-methyl-N-[2-oxo-2-[(2,2,2-trifluoroethyl)amino]ethyl]-benzamide and has the following structure:

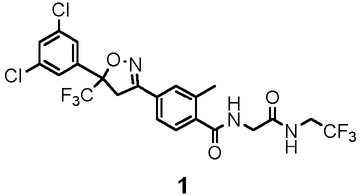

Fluralaner, acts as an "ectoparasiticide". Fluralaner kills fleas, ticks and mites that have ingested the dog's or cat's blood by acting on their nervous system. It blocks the normal movement of charged chloride particles (ions) in and out of nerve cells, especially those associated with gamma-aminobutyric acid (GABA) and glutamate, two substances that convey messages between nerves (neurotransmitters). This results in uncontrolled activity of the nervous system and the paralysis and death of the parasites.

Fluralaner is available for oral (chewable tablets) and topical (spot-on) administration to dogs, for topical administration (spot-on) to cats, and for oral administration (additive for drinking water) to chickens. The spot-on solution contains 28 g of the active substance Fluralaner per 100 ml of solution (28% w/v). The excipients in the formulation are dimethylacetamide (DMA), glycofurol, diethyltoluamide (DEET), and acetone. Five strengths are available for dog bodyweight ranges between 2 kg and 56 kg and three strengths are available for cat bodyweight ranges between 1.2 kg and 12.5 kg; all strengths are made from identical bulk solution containing 28% w/v Fluralaner.

The chewable tablets comprise in addition to active substance the excipients sucrose, maize starch, sodium lauryl sulphate, magnesium stearate, aspartame, glycerol, soya bean oil (refined), macrogol 3350, and disodium pamoate monohydrate.

WO 2005/085216 discloses in synthetic example 214 a process for the synthesis of Fluralaner according to the following scheme:

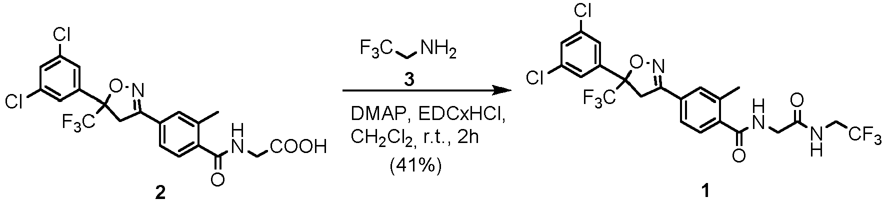

In a solution of 1.00 g of N-[4-[5-(3,5-dichlorophenyl)-5-trifluoromethyl-4,5-dihydroisoxazol-3-yl]-2-methylbenzoyl]glycine (2) in 30 ml of dichloromethane, 0.65 g of 1-[3-(diethylamino)propyl]-3-ethylcarbodiimide hydrochloride (EDC·HCl) was added, and stirred at room temperature for 15 minutes, then 0.40 g of 2,2,2-trifluoroethylamine and 0.40 g of 4-(N,N-dimethylamino)pyridine (DMAP) were added and stirred at the same temperature for further 2 hours. After the completion of the reaction, the solvent was distilled off under reduced pressure, the residue was purified with silica gel column chromatography that was eluted with ethyl acetate-hexane (1:3), and then crystallized from hexane to obtain 0.48 g of the aimed product as white crystal. Thus, the yields reported are low, only about 41%.

WO 2010/005048 discloses a first process for the synthesis of Fluralaner according to the following scheme:

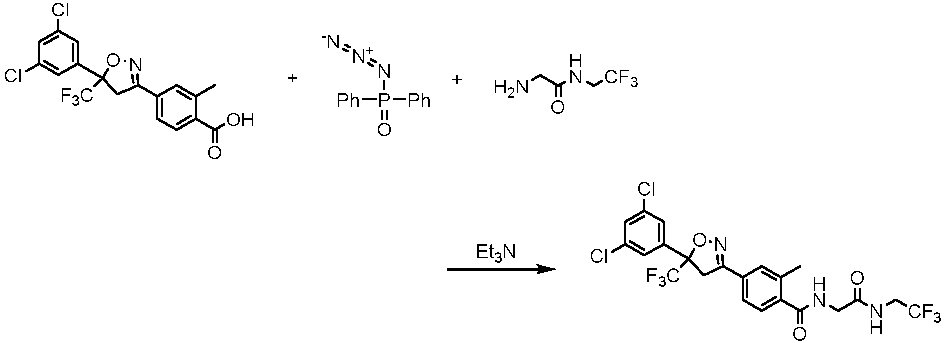

At the end of the reaction there is no isolation of the product, only the HPLC method assignation of the reaction mixture is used.

Further, WO 2010/005048 discloses a second process for the synthesis of Fluralaner which is one step synthesis from bromo intermediate to Fluralaner according to the following scheme:

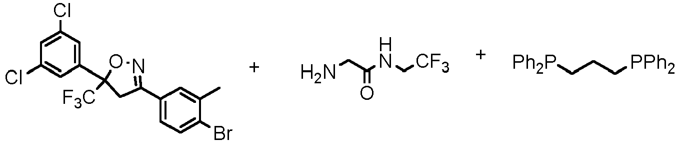

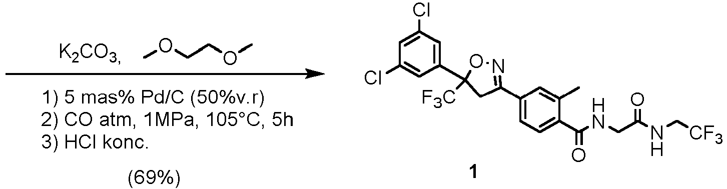

Furthermore, WO 2010/005048 discloses a second process for the synthesis of Fluralaner according to the following scheme:

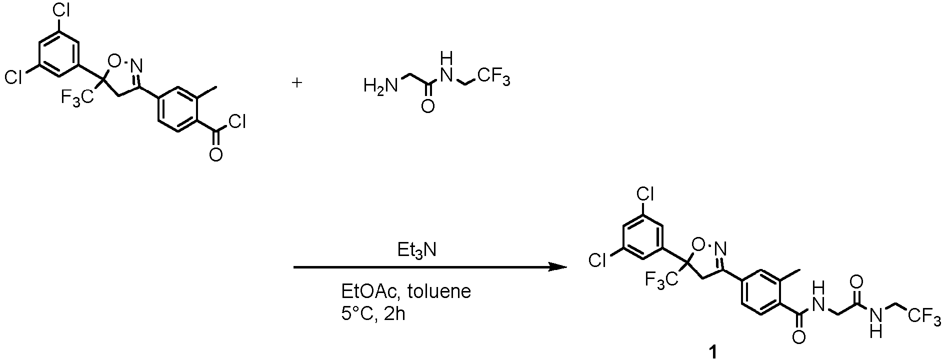

Although various approaches for preparing Fluralaner from various intermediates are known from the prior art, there still remains a need for improved procedures for preparing Fluralaner. There is a demand to make Fluralaner available at high yield and high purity so that synthesis is efficient and no complex and laborious work up procedures are necessary. Moreover, the procedures for preparing Fluralaner should allow for production on industrial scales.

S. Wangngae et al., RCS Adv. 2005, 5, 25789-25793 relates to the significance of reagent addition sequence in the amidation of carboxylic acids mediated by PPh₃ and I₂.

E. Valeur et al., Chem. Soc. Rev., 2009. 38, 606-631 critically review amide bond formation mediated by coupling reagents.

It is an object of the invention to provide processes for the chemical synthesis of Fluralaner having advantages compared to the processes of the prior art.

This object has been achieved by the subject-matter of the patent claims.

Unexpectedly, the process of the present invention considerably improves the known approaches for preparing Fluralaner. It has been surprisingly found that the low yield of the process known from the prior art, e.g. from WO 2005/085216, is due to an unwanted side reaction which according to the invention can be suppressed by defined order of addition of the reagents. It has been unexpectedly found that according to prior art process up to 50% of the product material comprises a "dimeric impurity" of the following formula: 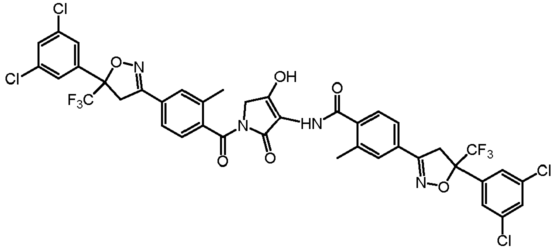 which is a product of an unwanted side reaction.

It has been surprisingly found that a slight modification of the process known from the prior art provides for improved yield of Fluralaner, easy purification, and suitability of the process for industrial production. The present invention provides process which minimizes the formation of this dimeric impurity, thereby significantly improved yields and minimizes need for purification.

Without wishing to be bound to any theory, it is assumed that the order of addition of specific reagents to the reaction mixture surprisingly reduces the formation of side products, especially of the dimeric impurity, and thereby essentially contributes to the preparation of Fluralaner in high yields and with high purity (e.g. with a purity of more than 98%). The order of addition of reagents therefore provides the advantage that the formation of side product can be avoided or minimized and that purification procedures, especially complex, material-intensive, laborious and time-consuming purification procedures, such as column chromatography, may be avoided.

The aspects, advantageous features and preferred embodiments of the present invention summarized hereinafter, respectively, alone or in combination with one another, further contribute to solving the problem of the present invention.

A first aspect of the invention relates to a process for preparing Fluralaner of formula (1), 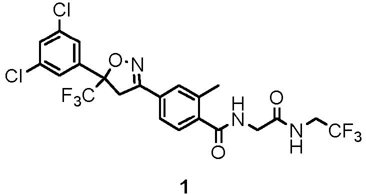 said process comprising the steps of:
(a) providing a composition comprising
   - a solvent;
   - (4-(5-(3,5-dichlorophenyl)-5-(trifluoromethyl)-4,5-dihydroisoxazol-3-yl)-2-methylbenzoyl)-glycine (2) 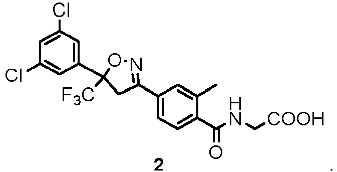
   - a catalyst which is either (i) a base or (ii) a N-hydroxyl compound; and
   - either (i) 2,2,2-trifluoroethylamine (or a salt thereof, for example hydrochloride, etc.) or (ii) a carboxylic acid activating agent, preferably a carbodiimide;
(b) adding to the composition provided in step (a) either (i) a carboxylic acid activating agent, preferably a carbodiimide, or (ii) 2,2,2-trifluoroethylamine (or a salt thereof, for example hydrochloride, etc.);
(c) allowing the composition obtained in step (b) to react thereby synthesizing Fluralaner (1); and
(d) optionally, recovering Fluralaner (1) from the composition obtained in step (c).

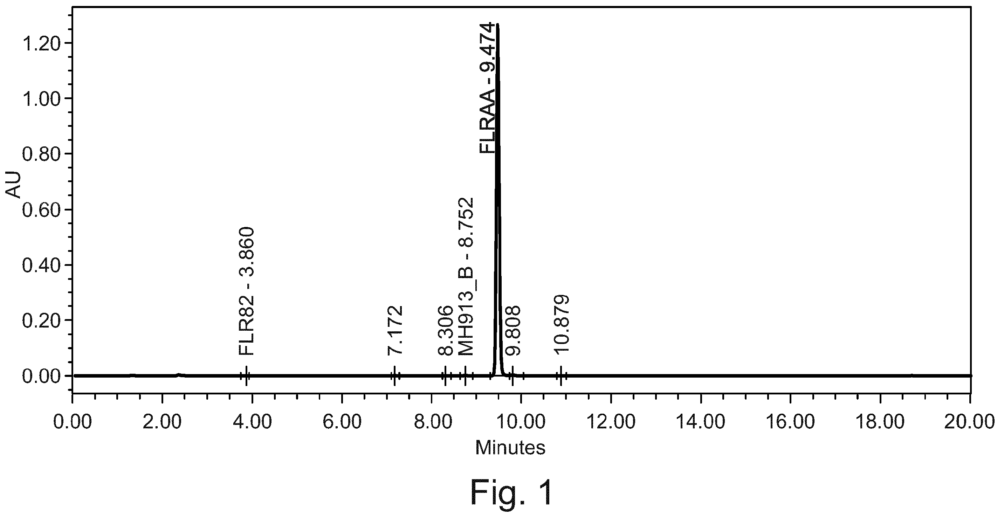

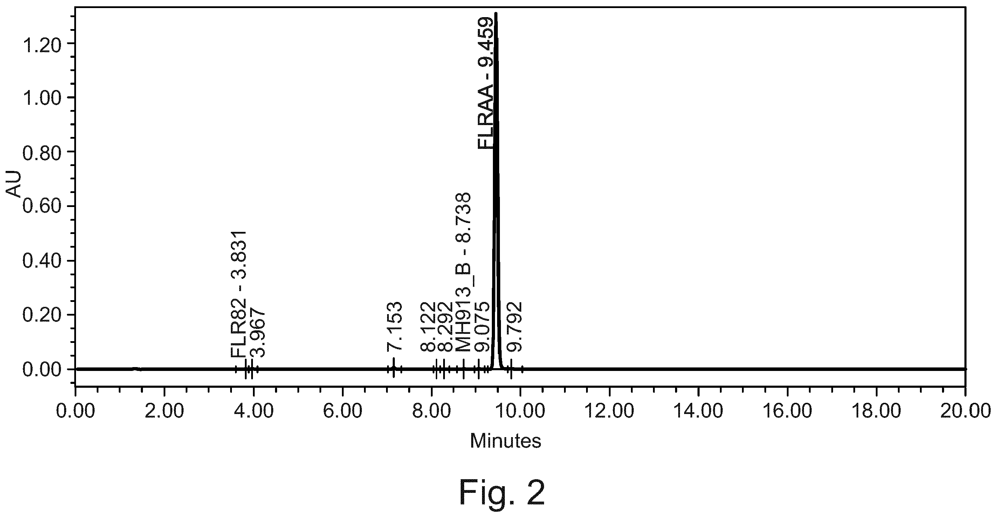

Figure 1 shows a chromatogram of the crude reaction product obtained in Example 1. Figure 2 shows a chromatogram of the crude reaction product obtained in Example 2. In each case, FLRAA (retention time about 9.5 minutes) is Fluralaner; FLE82 (retention time about 3.8 minutes) is the starting compound (2); and MH913 (retention time about 8.7 minutes) is the dimeric impurity.

The 2,2,2-trifluoroethylamine may be employed in form of the free base or as a salt thereof. Suitable salts of 2,2,2-trifluoroethylamine include for instance the hydrochloride and hydrobromide.

The process according to the invention encompasses two variants, variant (i) and variant (ii). Both variants (i) and (ii) have in common that in contrast to synthetic example 214 of WO 2005/085216, the (4-(5-(3,5-dichlorophenyl)-5-(trifluoromethyl)-4,5-dihydroisoxazol-3-yl)-2-methylbenzoyl)glycine (2) is not allowed to react with the carboxylic acid activating agent in absence of the catalyst and the 2,2,2-trifluoroethylamine (or a salt thereof, for example hydrochloride, etc.), respectively.

The composition provided in step (a) is preferably stirred for at least 10 minutes, preferably for at least 15 minutes before addition of either (i) a carboxylic acid activating agent, preferably a carbodiimide, or (ii) 2,2,2-trifluoroethylamine (or a salt thereof, for example hydrochloride, etc.) in step (b).

When the carboxylic acid activating agent is the last reagent that is added to the composition (variant i), it is preferably added to the composition provided in step (a) after the composition is stirred for at least 5 minutes, preferably for at least 10 minutes, more preferably for at least15 minutes. In this regard, the reagents comprised in the composition provided in step (a) are the compound of formula (2), the 2,2,2-trifluoroethylamine (or a salt thereof, for example hydrochloride, etc.) and the base as a catalyst.

When the carboxylic acid activating agent is not the last reagent that is added to the composition (variant ii), the missing reagents are preferably added to the composition provided in step (a) after the composition is stirred for at least 15 minutes, preferably for at least 30 minutes, more preferably for at least 60 minutes. In this regard, the reagents comprised in the composition provided in step (a) are the compound of formula (2), the carboxylic acid activating agent, and the N-hydroxyl compound as a catalyst.

Preferably, all the above reagents are added to the solvent within a relatively short period of time, preferably in less than 15 minutes, more preferably at most 10 minutes, still more preferably at most 5 minutes.

According to variant (i) of the process according to the invention, the composition provided in step (a) comprises the solvent; the (4-(5-(3,5-dichlorophenyl)-5-(trifluoromethyl)-4,5-dihydroisoxazol-3-yl)-2-methylbenzoyl)glycine (2); the catalyst, preferably a base; and the 2,2,2-trifluoroethylamine (or a salt thereof, for example hydrochloride, etc.); but not yet the carboxylic acid activating agent, preferably a carbodiimide. According to this variant (i) of the process according to the invention, the carboxylic acid activating agent, preferably a carbodiimide, is subsequently added in step (b) to the composition provided in step (a).

According to variant (i) of the process according to the invention, preferably the entire amount of 2,2,2-trifluoroethylamine (or a salt thereof, for example hydrochloride, etc.) that is employed in the process is contained in the composition provided in step (a), whereas preferably the entire amount of the carboxylic acid activating agent that is employed in the process is added in step (b).

According to variant (i) of the process according to the invention, in step (a) the solvent, the compound of formula (2), the 2,2,2-trifluoroethylamine (or a salt thereof, for example hydrochloride, etc.), and the catalyst are admixed with one another before the carboxylic acid activating agent is added thereto in subsequent step (b).

According to variant (i) of the process according to the invention, the order of admixing the solvent, the compound of formula (2), the 2,2,2-trifluoroethylamine (or a salt thereof, for example hydrochloride, etc.), and the catalyst with one another in step (a) is not particularly limited.

Preferred orders of admixing (A) the solvent, (B) the compound of formula (2), (C) the 2,2,2-trifluoroethylamine (or a salt thereof, for example hydrochloride, etc.), and (D) the catalyst with one another are selected from the group consisting of (A)(B)(C)(D), (A)(B)(D)(C), (A)(C)(B)(D), (A)(C)(D)(B), (A)(D)(B)(C), (A)(D)(C),(B), (B)(A)-(C)(D), (B)(A)(D)(C), (B)(C)(A)(D), (B)(C)(D)(A), (B)(D)(A)(C), (B)(D)(C)(A), (C)(A)(B)(D), (C)(A)(D)(B), (C)(B)(A)(D), (C)(B)(D)(A), (C)(D)(A)(B), (C)(D)(B)(A), (D)(A)-(B)(C), (D)(A)-(C)(B), (D)(B)(A)(C), (D)(B)(C)(A), (D)(C)(A)(B), and (D)(C)(B)(A).

It is also contemplated that two or more of (A) the solvent, (B) the compound of formula (2), (C) the 2,2,2-trifluoroethylamine (or a salt thereof, for example hydrochloride, etc.), and (D) the catalyst are admixed with one another simultaneously, and/or that any of (A) the solvent, (B) the compound of formula (2), (C) the 2,2,2-trifluoroethylamine (or a salt thereof, for example hydrochloride, etc.), and (D) the catalyst is divided into two or more portions which are admixed with the other reagents or portions thereof in any order.

In a preferred embodiment of variant (i) of the process according to the invention, step (a) comprises the sub-steps of:
(a-1) providing a composition comprising the solvent and the (4-(5-(3,5-dichlorophenyl)-5-(trifluoromethyl)-4,5-dihydroisoxazol-3-yl)-2-methylbenzoyl)glycine (2); and
(a-2) adding to the composition provided in sub-step (a-1) the 2,2,2-trifluoroethylamine (or a salt thereof, for example hydrochloride, etc.) and the catalyst, preferably the base; wherein the 2,2,2-trifluoroethylamine and the catalyst, preferably the base, may be added to the composition sequentially in any order or simultaneously or any partial combination thereof.

According to variant (ii) of the process according to the invention, the composition provided in step (a) comprises the solvent; the (4-(5-(3,5-dichlorophenyl)-5-(trifluoromethyl)-4,5-dihydroisoxazol-3-yl)-2-methylbenzoyl)glycine (2); the catalyst, preferably a N-hydroxyl compound; and the carboxylic acid activating agent, preferably a carbodiimide; but not yet the 2,2,2-trifluoroethylamine (or a salt thereof, for example hydrochloride, etc.). According to this variant (ii) of the process according to the invention, the 2,2,2-trifluoroethylamine (or a salt thereof, for example hydrochloride, etc.) is subsequently added in step (b) to the composition provided in step (a).

According to variant (ii) of the process according to the invention, in step (a) the solvent, the compound of formula (2), the carboxylic acid activating agent, and the catalyst are admixed with one another before the 2,2,2-trifluoroethylamine (or a salt thereof, for example hydrochloride, etc.) is added thereto in subsequent step (b).

According to variant (ii) of the process according to the invention, preferably the entire amount of 2,2,2-trifluoroethylamine (or a salt thereof, for example hydrochloride, etc.) that is employed in the process is added in step (b), whereas preferably the entire amount of the carboxylic acid activating agent that is employed in the process is contained in the composition provided in step (a).

According to variant (ii) of the process according to the invention, the order of admixing the solvent, the compound of formula (2), the carboxylic acid activating agent, and the catalyst with one another in step (a) is not particularly limited. However, preferably the solvent, the compound of formula (2), and the carboxylic acid activating agent are not admixed with one another in the absence of the catalyst.

Preferred orders of admixing (A) the solvent, (B) the compound of formula (2), (C) the carboxylic acid activating agent, and (D) the catalyst with one another are selected from the group consisting of (A)(B)(C)(D), (A)(B)(D)(C), (A)(C)(B)(D), (A)(C)(D)(B), (A)(D)(B)(C), (A)(D)-(C)(B), (B)(A)(C)(D), (B)(A)(D)(C), (B)(C)(A)(D), (B)(C)(D)(A), (B)(D)(A)(C), (B)(D)(C)(A), (C)(A)(B)(D), (C)(A)(D)(B), (C)(B)(A)(D), (C)(B)(D)(A), (C)(D)(A)(B), (C)(D)(B)(A), (D)(A)-(B)(C), (D)(A)(C)(B), (D)(B)(A)(C), (D)(B)(C)(A), (D)(C)(A)(B), and (D)(C)(B)(A); more preferably from (A)(B)(D)(C), (A)(C)(D)(B), (A)(D)(B)(C), (A)(D)(C),(B), (B)(A)(D)(C), (B)(C)(D)(A), (B)(D)(A)(C), (B)(D)(C)(A), (C)(A)(D)(B), (C)(B)(D)(A), (C)(D)(A)(B), (C)(D)(B)(A), (D)(A)(B)(C), (D)(A)(C)(B), (D)(B)(A)(C), (D)(B)(C)(A), (D)(C)(A)(B), and (D)(C)(B)(A).

It is also contemplated that two or more of (A) the solvent, (B) the compound of formula (2), (C) the carboxylic acid activating agent, and (D) the catalyst are admixed with one another simultaneously, and/or that any of (A) the solvent, (B) the compound of formula (2), (C) the carboxylic acid activating agent, and (D) the catalyst is divided into two or more portions which are admixed with the other reagents or portions thereof in any order.

In a preferred embodiment of variant (ii) of the process according to the invention, step (a) comprises the sub-steps of:
(a-1) providing a composition comprising the solvent and the (4-(5-(3,5-dichlorophenyl)-5-(trifluoromethyl)-4,5-dihydroisoxazol-3-yl)-2-methylbenzoyl)glycine (2); and
(a-2) adding to the composition provided in sub-step (a-1) the carboxylic acid activating agent, preferably the carbodiimide, and the catalyst, preferably the N-hydroxyl compound; wherein the carboxylic acid activating agent, preferably the carbodiimide, and the catalyst, preferably the N-hydroxysuccinimide, may be added to the composition sequentially in any order or simultaneously or any partial combination thereof.

The solvent is selected from the group consisting of acetonitrile, acetone, N,N-dimethylformamide, tetrahydrofuran, ethyl acetate, dichloromethane, and mixtures thereof. In particular, the solvent can essentially consist of or comprise dichloromethane. The solvent can for example comprise at least 50 wt.-%, preferably at least 95 wt.-% of dichloromethane, based on the total weight of the solvent.

The compound of formula (2), i.e. (4-(5-(3,5-dichlorophenyl)-5-(trifluoromethyl)-4,5-dihydroisoxazol-3-yl)-2-methylbenzoyl)glycine, also referred to as N-[4-[5-(3,5-dichlorophenyl)-5-trifluoromethyl-4,5-dihydroisoxazol-3-yl]-2-methylbenzoyl]glycine, is known from the prior art and can be prepared e.g. as described in prior art documents cited above.

The concentration of the intermediate of formula (2) in the composition is not particularly limited and can be for example within the range of from 1 to 100 mmol·l⁻¹, more preferably from 2 to 50 mmol·l⁻¹, still more preferably from 5 to 20 mmol·l⁻¹, and most preferably about 10 mmol.l⁻¹.

2,2,2-trifluoroethylamine (or a salt thereof, for example hydrochloride, etc.) is commercially available.

The concentration of the 2,2,2-trifluoroethylamine (or a salt thereof, for example hydrochloride, etc.) in the composition is not particularly limited and can be for example within the range of from 5 to 320 mmol·l⁻¹, more preferably from 10 to 160 mmol·l⁻¹, still more preferably from 20 to 80 mmol·l⁻¹, and most preferably about 40 mmol·l⁻¹.

Suitable carboxylic acid activating agents are known to the skilled person. According to the invention, the carboxylic acid activating agents transiently react with the carboxyl group of compound (2). This reaction typically involves transient formation of reactive intermediates that subsequently react with the amino group of 2,2,2-trifluoroethylamine (or a salt thereof, for example hydrochloride, etc.), or that subsequently react with other reactants to form other reactive intermediates that in turn subsequently react with the amino group of 2,2,2-trifluoroethylamine (or a salt thereof, for example hydrochloride, etc.). Typical reactive intermediates include but are not limited to carboxylic halides (chlorides, fluorides), carboxylic azides, symmetrical or mixed anhydrides or reaction products with carbodiimides.

The carboxylic acid activating agent is selected from
- carbodiimides; preferably selected from N-(3-dimethylaminopropyl)-N'-ethylcarbodiimide · HCl, also referred to as 1-ethyl-3-(3-dimethylaminopropyl)carbodiimide hydrochloride (EDC·HCl, EDAC·HCl, WSC·HCl), dicyclohexylcarbodiimide (DCC), N-cyclohexyl-N'-(β-[N-methylmorpholino]ethyl)carbodiimide (CMC) and diisopropylcarbodiimide (DIC);
- phosphonium-type reagents; preferably selected from benzotriazol-1-yloxy-tris(dimethylamino)-phosphonium hexafluorophosphate (BOP), benzotriazol-1-yloxy-tripyrrolidino-phosphonium hexafluorophosphate (PyBOP^{®}), bromo-tripyrrolidino-phosphonium hexafluorophosphate (PyBrOP^{®}), 7-aza-benzotriazol-1-yloxy-tripyrrolidinophosphonium hexafluorophosphate (PyAOP), ethyl cyano-(hydroxyimino)acetato-O2)-tri-(1-pyrrolidinyl)-phosphonium hexafluorophosphate (PyOxim), and 3-(diethoxy-phosphoryloxy)-1,2,3-benzo[d] triazin-4(3H)-one (DEPBT);
- aminium/uronium-imonium-type reagents; preferably selected from 2-(1H-benzotriazol-1-yl)-N,N,N',N'-tetramethylaminium tetrafluoroborate/hexafluorophosphate (TBTU/HBTU), 2-(6-chloro-1H-benzotriazol-1-yl)-N,N,N',N'-tetramethylaminium hexafluorophosphate (HCTU), N-[(5-chloro-1H-benzotriazol-1-yl)-dimethylamino-morpholino]-uronium hexafluorophosphate N-oxide (HDMC), 2-(7-aza-1H-benzotriazol-1-yl)-N,N,N',N'-tetramethylaminium tetrafluoroborate/- hexafluorophosphate (TATU/HATU), 1-[1-(cyano-2-ethoxy-2-oxoethylideneaminooxy)-dimethylamino-morpholino]-uronium hexafluorophosphate (COMU), 2-(1-oxy-pyridin-2-yl)-1,1,3,3-tetramethylisothiouronium tetrafluoroborate (TOTT), and tetramethylfluoroformamidinium hexafluorophosphate (TFFH).

The above carboxylic acid activating agents are all commercially available.

The concentration of the carboxylic acid activating agent in the composition is not particularly limited and can be for example within the range of from 10 to 640 mmol.l⁻¹, more preferably from 20 to 320 mmol·l⁻¹, still more preferably from 40 to 160 mmol·l⁻¹, and most preferably about 85 mmol.l⁻¹.

Suitable catalysts for the amide formation between the compound of formula (2) and 2,2,2-trifluoroethylamine (or a salt thereof, for example hydrochloride, etc.) by means of a carboxylic acid activating agent are known to the skilled person.

The catalyst generally lowers the activation enthalpy of the reaction but is not consumed in the course of the reaction.

In a preferred embodiment, the catalyst is a base, preferably 4-dimethylaminopyridine (DMAP). In another preferred embodiment, the catalyst is a N-hydroxyl compound, preferably N-hydroxysuccinimide (NHS). It is also possible that the catalyst comprises both, a base and a N-hydroxyl compound.

When the catalyst is a base, the base is preferably an organic base, more preferably selected from aliphatic amines, alicyclic amines and aromatic amines. The amines may be primary amines, secondary amines or tertiary amines. Preferably, the base is selected from the group consisting of 4-dimethylaminopyridine (DIMAP), triethanolamine, N,N-diisopropylethylamine (DIPEA), N-methyl-morpholine (NMM), 2-dimethylaminopyridine, 2,4,6-trimethylpyridine, piperidine and 4-(2-piperidinoethyl)pyridine. Preferably, the base is 4-dimethylaminopyridine (DMAP).

The above bases are all commercially available.

The concentration of the base in the composition is not particularly limited and can be for example within the range of from 10 to 1000 mmol·l⁻¹, more preferably from 25 to 400 mmol·l⁻¹, still more preferably from 50 to 200 mmol·l⁻¹, and most preferably about 110 mmol.l⁻¹.

When the catalyst is a N-hydroxyl compound, the N-hydroxyl compound is preferably selected from the group consisting of N-hydroxysuccinimide, N-hydroxy-benzotriazoles, N-hydroxy-benzotriazines, and 2-hydroxyimines (oximes). Preferably, the N-hydroxyl compound is selected from N-hydroxysuccinimide, also referred to as 1-hydroxypyrrolidine-2,5-dione (NHS, HOSu); 1-hydroxybenzotriazole (HOBt), optionally immobilized on a solid carrier such as HOBt-6-sulfonamidomethyl resin HCl; 1-hydroxy-7-aza-1H-benzotriazole (HOAt); hydroxy-3,4-dihydro-4-oxo-1,2,3-benzotriazine (HOOBt, HODhbt); and ethyl 2-cyana-2-(hydroximino)acetate (Oxyma Pure^{®}). Preferably, the N-hydroxyl compound is N-hydroxysuccinimide.

The above N-hydroxyl compounds are all commercially available.

The concentration of the N-hydroxyl compound, preferably N-hydroxysuccinimide, in the composition is not particularly limited and can be for example within the range of from 10 to 640 mmol·l⁻¹, more preferably from 20 to 320 mmol·l⁻¹, still more preferably from 40 to 160 mmol·l⁻¹, and most preferably about 80 mmol·l⁻¹.

The relative molar ratio of the compound of formula (2) and the 2,2,2-trifluoroethan-1-amine (or a salt thereof, for example hydrochloride, etc.) is not particularly limited. Preferably, the relative molar ratio is within the range of from 1:1 to 1:10, more preferably from 1:2 to 1:8, still more preferably from 1:3 to 1:7, and most preferably about 1:6. The relative molar ratio of the 2,2,2-trifluoroethan-1-amine (or a salt thereof, for example hydrochloride, etc.) and the compound of formula (2) is less than 15, preferably less than 14, more preferably less than 13, still more preferably less than 12, yet more preferably less than 11, even more preferably less than 10, most preferably less than 9, and in particular less than 8.

The relative molar ratio of the compound of formula (2) and the carboxylic acid activating agent is not particularly limited. Preferably, the relative molar ratio is within the range of from 1:0.5 to 1:5, more preferably from 1:0.8 to 1:1.5, still more preferably from 1:1.1 to 1:1.4, and most preferably about 1 : 1.2.

The temperature of the reaction in step (c) of the process according to the invention is not particularly limited. Preferably, step (c) is carried out at a temperature of above 5°C, more preferably in the range of from 10 to 50°C, still more preferably from 15 to 40°C, yet more preferably from 20 to 30°C.

Preferably, the process according to the invention provides an overall yield of Fluralaner (1) of at least 45%, more preferably at least 50%, still more preferably at least 55%, yet more preferably at least 60%, even more preferably at least 65%, and most preferably at least 70%.

In preferred embodiments of the process according to the invention,
- the catalyst is 4-(N,N-dimethylamino)pyridine (DMAP) or N-hydroxysuccinimide (NHS);
- the solvent is dichloromethane; and
- the carboxylic acid activating agent is 1-[3-(diethylamino)propyl]-3-ethylcarbodiimide hydrochloride (EDC·HCl).

Furthermore, carrying out the reaction of the present invention can advantageously contribute to minimizing impurities, such as in particular dimeric impurity of formula of formula

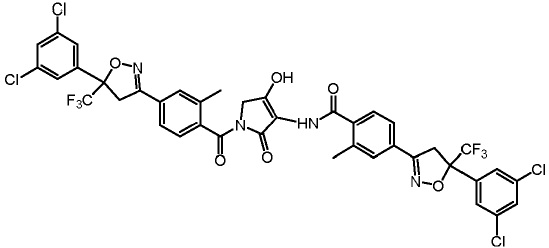

The content of dimeric impurity in the final product is preferably below 0.2 area% determined by HPLC, preferably below 0.15 area% determined by HPLC.

After step (c), Fluralaner of high purity can be recovered in optional step (d), preferably by isolating Fluralaner (directly) from the composition. Preferably, during the isolation of Fluralaner, firstly excess of carboxylic acid activating agents is decomposed, which is preferably done via quenching the reaction with acidic water solution (preferable 5 % HCl_{aq}). Secondly, extraction to organic phase is achieved with agitating the two phase mixture, subsequent separation of phases, and washing the water phase again with organic solvent in which the reaction was performed. The joined organic phases are preferably evaporated under reduced vacuum and a yellowish to white product is gained.

Fluralaner can be obtained as a product with high purity, e.g. all impurities can be HPLC < 0.30 area%, except the purity at retention time of about 1.035 minutes, which is a result of the impurity present in the starting material.

The purity of Fluralaner in general may be determined with the following HPLC method: column: Acquity CSH C18, 100x2.1mm, 1.7 µm, precolumn; flow-rate: 0.2ml/min; column temperature: 25°C, wavelength: UV 260 nm; mobile phase: eluent A: 5mM K₂HPO₄, Eluent B: 98% acetonitrile; gradient:

| Time (min) | % v/v A | % v/v B |
|---|---|---|
| 0-1 | 60 | 40 |
| 7-10 | 30 | 70 |
| 15-17 | 0 | 100 |
| 18-20 | 60 | 40 |

Sample preparation: Accurately weigh about 10 mg of sample and dissolve in 10 ml of methanol. Calculation: Use area per cent method. Do not integrate solvent peaks.

The following examples illustrate the invention.

### Example 1 - Synthesis of Fluralaner according to variant (i)

At room temperature (20-25°C) 10.0 g (21 mmol) (4-(5-(3,5-dichlorophenyl)-5-(trifluoromethyl)-4,5-dihydroisoxazol-3-yl)-2-methylbenzoyl)glycine were charged into 300 mL of dichloromethane, 12.5 g (126,2 mmol) 2,2,2-trifluoroethan-1-amine and 4 g (32.7 mmol) DMAP (4-dimethylaminopyridine) were added into the reactor vessel and agitated for at least 15 minutes to get white to yellowish suspension. Then 4.8 g (25 mmol) 1-ethyl-3-(3-dimethylaminopropyl)carbodiimide hydrochloride (EDC·HCl) were added and the mixture was agitated until reaction was finished according to HPLC analysis.

Then 300 mL of 5 % aqueous solution of hydrochloric acid were added, agitated for at least 15 minutes and the two phases were separated. Water phase was washed with 100 mL of dichloromethane and organic phase was separated. Both organic phases were combined and the solvent was evaporated to get white product (10.7 g) with HPLC purity above 95 area %. Reaction yield was 91.8 %.

The product from previous step was charged to the mixture of 19.3 mL of ethyl acetate and 64.2 mL of toluene. The mixture was agitated for some time and heated to 80°C to get yellow solution. If needed the solution was filtrated. The clear solution was cooled down to 0° - 5°C, agitated to precipitate the product which was filtered. The product was washed with the same mixture of ethyl acetate and toluene (3.9 mL : 12.8 mL) and sucked off well. The product was dried and after drying 8.8 g of white product with the HPLC purity above 99 area % was obtained. The residual content of compound (2) as determined by HPLC was less than 0.017 area%. The content of dimeric impurity as determined by HPLC was less than 0.14 area%.

Total yield for both steps was 75.5 %.

### Example 2 - Synthesis of Fluralaner according to variant (ii)

At room temperature (20-25°C) 10.0 g (21 mmol) (4-(5-(3,5-dichlorophenyl)-5-(trifluoromethyl)-4,5-dihydroisoxazol-3-yl)-2-methylbenzoyl)glycine were charged into 300 mL of dichloromethane, 2.7 g (23.4 mmol) NHS (hydroxysuccinimide, 1-hydroxypyrrolidine-2,5-dione) and 4.8 g (25 mmol) 1-ethyl-3-(3-dimethylaminopropyl)carbodiimide hydrochloride (EDC·HCl) were added and agitated. After some time (approximately 75 min) to the mixture 12.5 g (126.2 mmol) 2,2,2-trifluoroethan-1-amine were added and the mixture was agitated until reaction was finished according to HPLC analysis.

Then 300 mL of water were added to the mixture, agitated for at least 15 minutes and then was left to separate the phases. The organic phase was separated and the water phase was washed with 100 mL of dichloromethane. Both organic phases were combined and the solvent was evaporated o get dry yellowish product (11.1 g) in HPLC purity above 93 area %. Reaction yield was 95.2 %.

The product from previous step was charged to the mixture of 20 mL of ethyl acetate and 66.6 mL of toluene. The mixture was agitated for some time and heated to 80°C to get yellow solution. If needed the solution was filtrated. The clear solution was cooled down to 0°- 5°C and agitated to precipitate the product which was then filtered and washed using the same mixture of ethyl acetate and toluene (3.9 mL : 12.8 mL) and sucked off well. The product was dried and 8.6 g of white product with the HPLC purity above 98 area % were obtained.

The residual content of compound (2) as determined by HPLC was less than 0.14 area%. The content of dimeric impurity as determined by HPLC was less than 0.13 area%.

Total yield for both steps was 73.9 %.

## Claims

1. A process for preparing Fluralaner of formula (1), 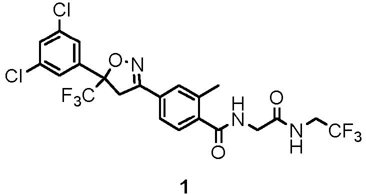 said process comprising the steps of:
(a) providing a composition comprising
- a solvent which is selected from the group consisting of acetonitrile, acetone, N,N-dimethylformamide, tetrahydrofuran, ethyl acetate, dichloromethane, and mixtures thereof;
- (4-(5-(3,5-dichlorophenyl)-5-(trifluoromethyl)-4,5-dihydroisoxazol-3-yl)-2-methylbenzoyl)glycine (2) 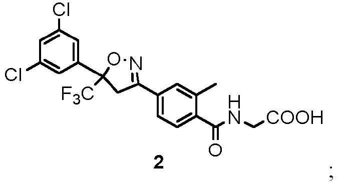
- a catalyst which is either (i) a base or (ii) a N-hydroxyl compound; and
- either (i) 2,2,2-trifluoroethylamine or a salt thereof, or (ii) a carboxylic acid activating agent which is selected from carbodiimides, phosphonium-type reagents, and aminium/uronium-imonium-type reagents;
(b) adding to the composition provided in step (a) either (i) a carboxylic acid activating agent which is selected from carbodiimides, phosphonium-type reagents, and aminium/uroniumimonium-type reagents, or (ii) 2,2,2-trifluoroethylamine or a salt thereof;
wherein the relative molar ratio of the 2,2,2-trifluoroethan-1-amine or a salt thereof and the compound of formula (2) is less than 15;
(c) allowing the composition obtained in step (b) to react thereby synthesizing Fluralaner (1); and
(d) optionally, recovering Fluralaner (1) from the composition obtained in step (c).

2. The process according to claim 1, wherein (i) the composition provided in step (a) comprises 2,2,2-trifluoroethylamine or a salt thereof, and wherein in step (b) the carboxylic acid activating agent is added; preferably the entire amount of 2,2,2-trifluoroethylamine or a salt thereof that is employed in the process is contained in the composition provided in step (a), and wherein the entire amount of the carboxylic acid activating agent that is employed in the process is added in step (b).

3. The process according to claim 1 or 2, wherein the composition provided in step (a) comprises the solvent, the (4-(5-(3,5-dichlorophenyl)-5-(trifluoromethyl)-4,5-dihydroisoxazol-3-yl)-2-methylbenzoyl)glycine (2), the catalyst, and the 2,2,2-trifluoroethylamine or a salt thereof, but not yet the carboxylic acid activating agent; and wherein the carboxylic acid activating agent is subsequently added in step (b) to the composition provided in step (a);
and step (a) comprises the sub-steps of:
(a-1) providing a composition comprising the solvent and the (4-(5-(3,5-dichlorophenyl)-5-(trifluoromethyl)-4,5-dihydroisoxazol-3-yl)-2-methylbenzoyl)glycine (2); and
(a-2) adding to the composition provided in sub-step (a-1) the 2,2,2-trifluoroethylamine or a salt thereof and the catalyst.

4. The process according to claim 1, wherein (ii) the composition provided in step (a) comprises the carboxylic acid activating agent, and wherein in step (b) 2,2,2-trifluoroethylamine or a salt thereof is added; preferably the entire amount of 2,2,2-trifluoroethylamine or a salt thereof that is employed in the process is added in step (b), and wherein the entire amount of the carboxylic acid activating agent that is employed in the process is contained in the composition provided in step (a).

5. The process according to claim 1 or 4, wherein the composition provided in step (a) comprises the solvent, the (4-(5-(3,5-dichlorophenyl)-5-(trifluoromethyl)-4,5-dihydroisoxazol-3-yl)-2-methylbenzoyl)glycine (2), the catalyst, and the carboxylic acid activating agent, but not yet the 2,2,2-trifluoroethylamine or a salt thereof; and wherein the 2,2,2-trifluoroethylamine or a salt thereof is subsequently added in step (b) to the composition provided in step (a);
and step (a) comprises the sub-steps of:
(a-1) providing a composition comprising the solvent and the (4-(5-(3,5-dichlorophenyl)-5-(trifluoromethyl)-4,5-dihydroisoxazol-3-yl)-2-methylbenzoyl)glycine (2); and
(a-2) adding to the composition provided in sub-step (a-1) the carboxylic acid activating agent and the catalyst.

6. The process according to any of the preceding claims, wherein the catalyst is a base which is selected from the group consisting of 4-dimethylaminopyridine, triethanolamine, N,N-diisopropylethylamine, N-methyl-morpholine, 2-dimethylaminopyridine, 2,4,6-trimethylpyridine, piperidine and 4-(2-piperidinoethyl)pyridine; preferably 4-dimethylaminopyridine.

7. The process according to any of claims 1 to 6, wherein the catalyst is a N-hydroxyl compound which is selected from the group consisting of N-hydroxysuccinimide, N-hydroxy-benzotriazoles, N-hydroxy-benzotriazines, and 2-hydroxyimines; preferably N-hydroxysuccinimide.

8. The process according to any of the preceding claims, wherein the solvent is dichloromethane.

9. The process according to any of the preceding claims, wherein the carboxylic acid activating agent is a carbodiimide selected from 1-ethyl-3-(3-dimethylaminopropyl)carbodiimide hydrochloride, dicyclohexylcarbodiimide, N-cyclohexyl-N'-(β-[N-methylmorpholino]ethyl)carbodiimide, and diisopropylcarbodiimide; preferably 1-ethyl-3-(3-dimethylaminopropyl)carbodiimide hydrochloride.

10. The process according to any of the preceding claims, wherein the relative molar ratio of the 2,2,2-trifluoroethan-1-amine or a salt thereof and the compound of formula (2) is less than 14.

11. The process according to any of the preceding claims, wherein the relative molar ratio of the compound of formula (2) and the 2,2,2-trifluoroethan-1-amine or a salt thereof is within the range of from 1:1 to 1:10.

12. The process according to any of the preceding claims, wherein the relative molar ratio of the compound of formula (2) and the carboxylic acid activating agent is within the range of from 1:0.5 to 1:5.

13. The process according to any of the preceding claims, wherein step (c) is carried out at a temperature within the range of from 10 to 50 °C.

14. The process according to any of the preceding claims, wherein
- the catalyst is 4-(N,N-dimethylamino)pyridine (DMAP) or N-hydroxysuccinimide (NHS);
- the solvent is dichloromethane; and
- the carboxylic acid activating agent is 1-[3-(diethylamino)propyl]-3-ethylcarbodiimide hydrochloride (EDC·HCl).

15. The process according to any of the preceding claims, which provides an overall yield of Fluralaner (1) of at least 45%, preferably at least 50%.

## Patentansprüche

1. Ein Verfahren zur Herstellung von Fluralaner der Formel (1), 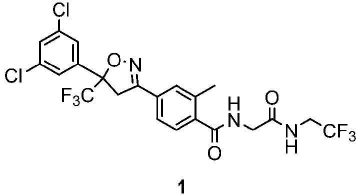 wobei das Verfahren die folgenden Schritte umfasst:
(a) Bereitstellen einer Zusammensetzung, die umfasst
- ein Lösungsmittel, das aus der Gruppe ausgewählt ist, die aus Acetonitril, Aceton, N,N-Dimethylformamid, Tetrahydrofuran, Ethylacetat, Dichlormethan und Mischungen davon besteht;
- (4-(5-(3,5-Dichlorphenyl)-5-(trifluormethyl)-4,5-dihydroisoxazol-3-yl)-2-methylbenzoyl)glycin (2) 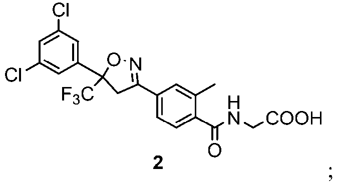
- einen Katalysator, der entweder (i) eine Base oder (ii) eine N-Hydroxylverbindung ist; und
- entweder (i) 2,2,2-Trifluorethylamin oder ein Salz davon oder (ii) ein Carbonsäure-Aktivierungsmittel, das aus Carbodiimiden, Phosphonium-Reagenzien und Aminium/Uronium-Imonium-Reagenzien ausgewählt ist;
(b) Hinzufügen zu der in Schritt (a) bereitgestellten Zusammensetzung entweder (i) eines Carbonsäureaktivierungsmittels, das aus Carbodiimiden, Phosphonium-Reagenzien und Aminium/Uronium-Imonium-Reagenzien ausgewählt ist, oder (ii) von 2,2,2-Trifluorethylamin oder einem Salz davon;
wobei das relative Molverhältnis von 2,2,2-Trifluorethan-1-amin oder einem Salz davon und der Verbindung der Formel (2) weniger als 15 beträgt;
(c) Reagierenlassen der in Schritt (b) erhaltenen Zusammensetzung, wodurch Fluralaner (1) synthetisiert wird; und
(d) gegebenenfalls Gewinnen von Fluralaner (1) aus der in Schritt (c) erhaltenen Zusammensetzung.

2. Das Verfahren nach Anspruch 1, wobei (i) die in Schritt (a) bereitgestellte Zusammensetzung 2,2,2-Trifluorethylamin oder ein Salz davon umfasst und wobei in Schritt (b) das Carbonsäure-Aktivierungssmittel zugegeben wird; vorzugsweise ist die gesamte Menge an 2,2,2-Trifluorethylamin oder einem Salz davon, die in dem Verfahren verwendet wird, in der in Schritt (a) bereitgestellten Zusammensetzung enthalten, und wobei die gesamte Menge des Carbonsäure-Aktivierungsmittels, die in dem Verfahren verwendet wird, in Schritt (b) zugegeben wird.

3. Das Verfahren nach Anspruch 1 oder 2, wobei die in Schritt (a) bereitgestellte Zusammensetzung das Lösungsmittel, das (4-(5-(3,5-Dichlorphenyl)-5-(trifluormethyl)-4,5-dihydroisoxazol-3-yl)-2-methylbenzoyl)glycin (2), den Katalysator und das 2,2,2-Trifluorethylamin oder ein Salz davon, jedoch noch nicht das Carbonsäure-Aktivierungsmittel umfasst; und wobei das Carbonsäure-Aktivierungsmittel anschließend in Schritt (b) zu der in Schritt (a) bereitgestellten Zusammensetzung hinzugefügt wird;
und Schritt (a) die folgenden Teilschritte umfasst:
(a-1) Bereitstellen einer Zusammensetzung, die das Lösungsmittel und das (4-(5-(3,5-Dichlorphenyl)-5-(trifluormethyl)-4,5-dihydroisoxazol-3-yl)-2-methylbenzoyl)glycin (2) umfasst; und
(a-2) Zugeben des 2,2,2-Trifluorethylamins oder eines Salzes davon und des Katalysators zu der in Teilschritt (a-1) bereitgestellten Zusammensetzung.

4. Das Verfahren nach Anspruch 1, wobei (ii) die in Schritt (a) bereitgestellte Zusammensetzung das Carbonsäure-Aktivierungsmittel umfasst und wobei in Schritt (b) 2,2,2-Trifluorethylamin oder ein Salz davon zugegeben wird; vorzugsweise wird die gesamte Menge an 2,2,2-Trifluorethylamin oder einem Salz davon, die in dem Verfahren verwendet wird, in Schritt (b) zugegeben, und wobei die gesamte Menge des Carbonsäureaktivierungsmittels, die in dem Verfahren verwendet wird, in der in Schritt (a) bereitgestellten Zusammensetzung enthalten ist.

5. Das Verfahren nach Anspruch 1 oder 4, wobei die in Schritt (a) bereitgestellte Zusammensetzung das Lösungsmittel, das (4-(5-(3,5-Dichlorphenyl)-5-(trifluormethyl)-4,5-dihydroisoxazol-3-yl)-2-methylbenzoyl)glycin (2), den Katalysator und das Carbonsäureaktivierungsmittel, jedoch noch nicht das 2,2,2-Trifluorethylamin oder ein Salz davon enthält; und wobei das 2,2,2-Trifluorethylamin oder ein Salz davon anschließend in Schritt (b) zu der in Schritt (a) bereitgestellten Zusammensetzung hinzugefügt wird;
und Schritt (a) umfasst die folgenden Teilschritte:
(a-1) Bereitstellen einer Zusammensetzung, die das Lösungsmittel und das (4-(5-(3,5-Dichlorphenyl)-5-(trifluormethyl)-4,5-dihydroisoxazol-3-yl)-2-methylbenzoyl)glycin (2) umfasst; und
(a-2) Zugeben des Carbonsäure-Aktivierungsmittels und des Katalysators zu der in Teilschritt (a-1) bereitgestellten Zusammensetzung.

6. Das Verfahren nach einem der vorstehenden Ansprüche, wobei der Katalysator eine Base ist, die aus der Gruppe ausgewählt ist, die aus 4-Dimethylaminopyridin, Triethanolamin, N,N-Diisopropylethylamin, N-Methylmorpholin, 2-Dimethylaminopyridin, 2,4,6-Trimethylpyridin, Piperidin und 4-(2-Piperidinoethyl)pyridin ausgewählt ist; vorzugsweise 4-Dimethylaminopyridin.

7. Das Verfahren nach einem der Ansprüche 1 bis 6, wobei der Katalysator eine N-Hydroxylverbindung ist, die aus der Gruppe ausgewählt ist, die aus N-Hydroxysuccinimid, N-Hydroxybenzotriazolen, N-Hydroxybenzotriazinen und 2-Hydroxyiminen besteht; vorzugsweise N-Hydroxysuccinimid.

8. Das Verfahren nach einem der vorstehenden Ansprüche, wobei das Lösungsmittel Dichlormethan ist.

9. Das Verfahren nach einem der vorstehenden Ansprüche, wobei das Carbonsäure-Aktivierungsmittel ein Carbodiimid ist, ausgewählt aus 1-Ethyl-3-(3-dimethylaminopropyl)carbodiimid-Hydrochlorid, Dicyclohexylcarbodiimid, N-Cyclohexyl-N'-(β-[N-methylmorpholino]ethyl)carbodiimid und Diisopropylcarbodiimid, vorzugsweise 1-Ethyl-3-(3-dimethylaminopropyl)carbodiimidhydrochlorid.

10. Das Verfahren nach einem der vorstehenden Ansprüche, wobei das relative Molverhältnis von 2,2,2-Trifluorethan-1-amin oder einem Salz davon und der Verbindung der Formel (2) weniger als 14 beträgt.

11. Das Verfahren nach einem der vorstehenden Ansprüche, wobei das relative Molverhältnis der Verbindung der Formel (2) und des 2,2,2-Trifluorethan-1-amins oder eines Salzes davon im Bereich von 1:1 bis 1:10 liegt.

12. Das Verfahren nach einem der vorstehenden Ansprüche, wobei das relative Molverhältnis der Verbindung der Formel (2) und des Carbonsäure-Aktivierungsmittels im Bereich von 1:0,5 bis 1:5 liegt.

13. Das Verfahren nach einem der vorstehenden Ansprüche, wobei Schritt (c) bei einer Temperatur im Bereich von 10 bis 50 °C durchgeführt wird.

14. Das Verfahren nach einem der vorstehenden Ansprüche, wobei
- der Katalysator 4-(N,N-Dimethylamino)pyridin (DMAP) oder N-Hydroxysuccinimid (NHS) ist;
- das Lösungsmittel Dichlormethan ist; und
- das Carbonsäure-Aktivierungsmittel 1-[3-(Diethylamino)propyl]-3-ethylcarbodiimidhydrochlorid (EDC·HCl) ist.

15. Das Verfahren nach einem der vorstehenden Ansprüche, das eine Gesamtausbeute an Fluralaner (1) von mindestens 45 %, vorzugsweise mindestens 50 %, liefert.

## Revendications

1. Procédé de préparation de Fluralaner de formule (1), 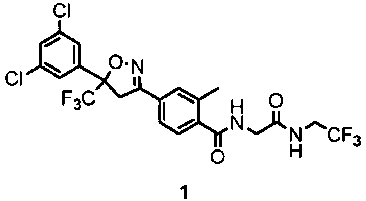 ledit procédé comprenant les étapes de :
(a) fourniture d'une composition comprenant
- un solvant qui est choisi dans le groupe constitué par l'acétonitrile, l'acétone, le N,N-diméthylformamide, le tétrahydrofurane, l'acétate d'éthyle, le dichlorométhane et leurs mélanges ;
- (4-(5-(3,5-dichlorophényl)-5-(trifluorométhyl)-4,5-dihydroisoxazol-3-yl)-2-méthylbenzoyl)glycine (2) 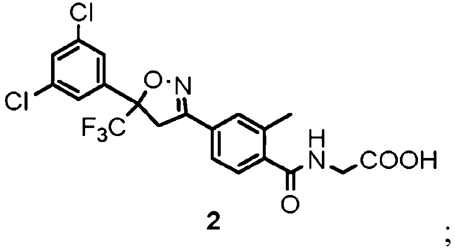
- un catalyseur qui est soit (i) une base, soit (ii) un composé N-hydroxyle ; et
- soit (i) la 2,2,2-trifluoroéthylamine ou un sel de celle-ci, soit (ii) un agent activateur d'acide carboxylique qui est choisi parmi les carbodiimides, les réactifs de type phosphonium et les réactifs de type aminium/uronium-imonium ;
(b) ajout à la composition fournie à l'étape (a) soit (i) d'un agent activateur d'acide carboxylique qui est choisi parmi les carbodiimides, les réactifs de type phosphonium et les réactifs de type aminium/uronium-imonium, ou (ii) de la 2,2,2-trifluoroéthylamine ou un sel de celle-ci ;
dans lequel le rapport molaire relatif de la 2,2,2-trifluoroéthan-1-amine ou d'un sel de celle-ci et du composé de formule (2) est inférieur à 15 ;
(c) permission à la composition obtenue à l'étape (b) de réagir, synthétisant ainsi le Fluralaner (1) ; et
(d) éventuellement, récupération du Fluralaner (1) à partir de la composition obtenue à l'étape (c).

2. Procédé selon la revendication 1, dans lequel (i) la composition fournie à l'étape (a) comprend la 2,2,2-trifluoroéthylamine ou un sel de celle-ci, et dans lequel l'agent activateur d'acide carboxylique est ajouté à l'étape (b) ; de préférence, la totalité de la 2,2,2-trifluoroéthylamine ou de son sel utilisée dans le procédé est contenue dans la composition fournie à l'étape (a), et dans lequel la totalité de l'agent activateur d'acide carboxylique utilisé dans le procédé est ajoutée à l'étape (b).

3. Procédé selon la revendication 1 ou 2, dans lequel la composition fournie à l'étape (a) comprend le solvant, la (4-(5-(3,5-dichlorophényl)-5-(trifluorométhyl)-4,5-dihydroisoxazol-3-yl)-2-méthylbenzoyl)glycine (2), le catalyseur et la 2,2,2-trifluoroéthylamine ou un sel de celle-ci, mais pas encore l'agent activateur d'acide carboxylique ; et dans lequel l'agent activateur d'acide carboxylique est ensuite ajouté à l'étape (b) à la composition fournie à l'étape (a) ;
et l'étape (a) comprend les sous-étapes suivantes :
(a-1) la fourniture d'une composition comprenant le solvant et le (4-(5-(3,5-dichlorophényl)-5-(trifluorométhyl)-4,5-dihydroisoxazol-3-yl)-2-méthylbenzoyl)glycine (2) ; et
(a-2) l'ajout à la composition fournie dans la sous-étape (a-1) de la 2,2,2-trifluoroéthylamine ou un sel de celle-ci et du catalyseur.

4. Procédé selon la revendication 1, dans lequel (ii) la composition fournie à l'étape (a) comprend l'agent activateur d'acide carboxylique, et dans lequel à l'étape (b) la 2,2,2-trifluoroéthylamine ou un sel de celle-ci est ajouté ; de préférence, la totalité de la 2,2,2-trifluoroéthylamine ou de son sel utilisée dans le procédé est contenue dans à l'étape (b), et dans lequel la totalité de l'agent activateur d'acide carboxylique utilisé dans le procédé est contenue dans la composition fournie à l'étape (a).

5. Procédé selon la revendication 1 ou 4, dans lequel la composition fournie à l'étape (a) comprend le solvant, la (4-(5-(3,5-dichlorophényl)-5-(trifluorométhyl)-4,5-dihydroisoxazol-3-yl)-2-méthylbenzoyl)glycine (2), le catalyseur et l'agent activateur d'acide carboxylique, mais pas encore la 2,2,2-trifluoroéthylamine ou un sel de celle-ci ; et dans lequel la 2,2,2-trifluoroéthylamine ou un sel de celle-ci est ensuite ajoutée à l'étape (b) à la composition fournie à l'étape (a) ;
et l'étape (a) comprend les sous-étapes suivantes :
(a-1) la fourniture d'une composition comprenant le solvant et le (4-(5-(3,5-dichlorophényl)-5-(trifluorométhyl)-4,5-dihydroisoxazol-3-yl)-2-méthylbenzoyl)glycine (2) ; et
(a-2) l'ajout à la composition fournie dans la sous-étape (a-1) de l'agent activateur d'acide carboxylique et du catalyseur.

6. Procédé selon l'une quelconque des revendications précédentes, dans lequel le catalyseur est une base choisie dans le groupe constitué par la 4-diméthylaminopyridine, la triéthanolamine, la N,N-diisopropyléthylamine, la N-méthylmorpholine, la 2-diméthylaminopyridine, la 2,4,6-triméthylpyridine, la pipéridine et la 4-(2-pipéridinoéthyl)pyridine ; de préférence la 4-diméthylaminopyridine.

7. Procédé selon l'une quelconque des revendications 1 à 6, dans lequel le catalyseur est un composé N-hydroxyle choisi dans le groupe constitué par le N-hydroxysuccinimide, les N-hydroxybenzotriazoles, les N-hydroxybenzotriazines et les 2-hydroxyimines ; de préférence le N-hydroxysuccinimide.

8. Procédé selon l'une quelconque des revendications précédentes, dans lequel le solvant est le dichlorométhane.

9. Procédé selon l'une quelconque des revendications précédentes, dans lequel l'agent activateur d'acide carboxylique est un carbodiimide choisi parmi le chlorhydrate de 1-éthyl-3-(3-diméthylaminopropyl)carbodiimide, le dicyclohexylcarbodiimide, le N-cyclohexyl-N'-(β-[N-méthylmorpholino]éthyl)carbodiimide et le diisopropylcarbodiimide ; de préférence le chlorhydrate de 1-éthyl-3-(3-diméthylaminopropyl)carbodiimide.

10. Procédé selon l'une quelconque des revendications précédentes, dans lequel le rapport molaire relatif de la 2,2,2-trifluoroéthan-1-amine ou d'un sel de celle-ci et du composé de formule (2) est inférieur à 14.

11. Procédé selon l'une quelconque des revendications précédentes, dans lequel le rapport molaire relatif de la formule (2) et de la 2,2,2-trifluoroéthan-1-amine ou d'un sel de celle-ci se situe dans une plage allant de 1:1 à 1:10.

12. Procédé selon l'une quelconque des revendications précédentes, dans lequel le rapport molaire relatif du composé de la formule (2) et l'agent activateur d'acide carboxylique se situe dans la plage allant de 1:0.5 à 1:5.

13. Procédé selon l'une quelconque des revendications précédentes, dans lequel l'étape (c) est réalisée à une température comprise entre 10 et 50 °C.

14. Procédé selon l'une quelconque des revendications précédentes, dans lequel
- le catalyseur est la 4-(N,N-diméthylamino)pyridine (DMAP) ou la N-hydroxysuccinimide (NHS) ;
- le solvant est le dichlorométhane ; et
- l'agent activateur d'acide carboxylique est le chlorhydrate de 1-[3-(diéthylamino)propyl]-3-éthylcarbodiimide (EDC·HCl).

15. Procédé selon l'une quelconque des revendications précédentes, qui fournit un rendement global de Fluralaner (1) d'au moins 45 %, de préférence d'au moins 50 %.
